# EUROPÄISCHE PATENTANMELDUNG

(11) **EP 1 568 782 A1**
(43) Veröffentlichungstag der Anmeldung: **31.08.2005**
(21) Anmeldenummer: 04004214.5
(22) Anmeldetag: 25.02.2004
(51) Int. Cl.: C12Q 1/37

(54) **Diagnose und Therapie von ADAMTS-13 assoziierten Erkrankungen**

(71) Anmelder: Clemens Bockmeyer, 07743 Jena (DE)
(72) Erfinder: Reinhart, Konrad, Prof. Dr. med., 07743 Jena (DE); Budde, Ulrich, Prof. Dr. med., 22607 Hamburg (DE); Claus, Ralf Alexander, Dr. rer.nat., 07747 Jena (DE); Brunkhorst, Frank Martin, Dr. med., 07743 Jena (DE); Kentouche, Karim, Dr. med., 07743 Jena (DE); Deigner, Hans-Peter, Dr. rer.nat., 68623 Lampertheim (DE); Bockmeyer, Clemens, 07743 Jena (DE)
(74) Vertreter: Schüssler, Andrea

(57) **Zusammenfassung**

Beschrieben werden Verfahren zur Diagnose von Entzündungen, Infektionen und/oder Gerinnungsstörungen, wobei eine Erniedrigung der Konzentration und/oder Aktivität von ADAMTS-13 diagnostisch relevant ist, sowie Kits zur Diagnose. Beschrieben werden auch entsprechende Therapieverfahren, die auf der Erhöhung der Konzentration und/oder Aktivität von ADAMTS-13 basieren.

## Beschreibung

Die vorliegende Erfindung betrifft Verfahren zur Diagnose von Entzündungen, Infektionen und/oder Gerinnungsstörungen, wobei eine Erniedrigung der Konzentration und/oder Aktivität von ADAMTS-13 diagnostisch relevant ist, sowie Kits zur Diagnose. Die vorliegende Erfindung betrifft auch entsprechende Therapieverfahren, die auf der Erhöhung der Konzentration und/oder Aktivität von ADAMTS-13 basieren.

Als Entzündungen (Inflammationen) werden im allgemeinen bestimmte physiologische Reaktionen eines Organismus auf verschiedenartige äußere Einwirkungen wie z. B. Verletzungen, Verbrennungen, Allergene, Infektionen durch Mikroorganismen wie Bakterien, Pilze und Viren, auf Fremdgewebe, die Abstoßungsreaktionen auslösen, oder auf bestimmte entzündungsauslösende endogene Zustände des Körpers, z. B. bei Autoimmunerkrankungen und Krebs, bezeichnet. Entzündungen können als harmlose, lokal begrenzte Reaktionen des Körpers auftreten, sind jedoch auch typische Merkmale ernster chronischer und/oder akuter Erkrankungen von einzelnen Geweben, Organ- und Gewebsteilen.

Lokale Entzündungen sind meist Teil einer adäquaten Immunreaktion des Körpers auf schädliche Einwirkungen und damit Teil des lebenserhaltenden Abwehrmechanismus des Organismus (Wirtsreaktion). Sobald Entzündungen jedoch Teil einer fehlgeleiteten Reaktion des Körpers auf bestimmte endogene Vorgänge wie z. B. bei Autoimmunerkrankungen und/oder chronischer Natur sind, oder wenn sie systemische Ausmaße erreichen, wie beim systemischen Inflammationsreaktions-Syndrom (Systemic Inflammatory Response Syndrome, SIRS) oder bei einer auf infektiöse Ursachen zurückzuführenden Sepsis, geraten die für Entzündungsreaktionen typischen physiologischen Vorgänge außer Kontrolle und entwickeln sich zum eigentlichen, häufig lebensbedrohlichen Krankheitsgeschehen.

Es ist heute bekannt, dass die Entstehung und der Verlauf insbesondere generalisierter entzündlicher Prozesse eng mit der Aktivierung und den daraus resultierenden Veränderungen des Blutgerinnungssystems vergesellschaftet sind. Die simultane Aktivierung der Immunantwort und des Blutgerinnungssystems in Folge einer Gewebsschädigung stellt ein phylogenetisch sehr altes Strategieelement zur Überlebenssicherung dar (Opal, S.M., Scand. J. Infect. Dis. 35 (2003), S. 545-554). Jüngste therapeutische Erfolge bei der Behandlung der schweren Sepsis und des septischen Schock beruhen u.a. auf der Anwendung rekombinant hergestellten, aktivierten Protein C, welches einen anti-inflammatorischen, anti-thrombotischen und profibrinolytischen Wirkmechanismus aufweist (Bernard et al., N.Engl.J.Med.344 (2001), 699-709). Weiterhin ist bekannt, dass die Entstehung und der Verlauf insbesondere generalisierter entzündlicher Prozesse von einer beträchtlichen Anzahl von Substanzen, die überwiegend proteinischer oder peptidischer Struktur sind, gesteuert werden bzw. von einem mehr oder weniger zeitlich begrenzten Auftreten bestimmter biologisch aktiver Moleküle begleitet sind. Zu den an Entzündungsreaktionen beteiligten endogenen Substanzen gehören insbesondere solche, welche zu den Zytokinen, Mediatoren, vasoaktiven Substanzen, Akutphase-Proteinen und/oder hormonellen Regulatoren, welche alle auch eine enzymatische Aktivität aufweisen können, gezählt werden. Die Wirtsreaktion stellt dabei eine komplexe physiologische Reaktion dar, an der sowohl das Entzündungs- sowie das Gerinnungssystem aktivierende endogene Substanzen (z. B. TNF-α bzw. Tissue factor) als auch inhibierende Substanzen (z. B. Interleukin-10 bzw. AntiThrombin) beteiligt sind.

Bei systemischen Entzündungen infektiöser Ätiologie wie im Fall einer Sepsis oder nicht-infektiöser Ätiologie wie im Rahmen eines kardiopulmonalen Bypasses weiten sich die entzündungsspezifischen Reaktionskaskaden unkontrolliert auf den gesamten Körper aus und werden dabei im Sinn einer überschießenden Immunantwort lebensbedrohlich. Da sich das Verständnis und damit auch die Definitionen von Sepsis und verwandten systemischen entzündlichen Erkrankungen in den vergangenen Jahren gewandelt haben, wird auf Reinhart et al., "Sepsis und septischer Schock", in: Intensivmedizin, Georg Thieme Verlag, Stuttgart, New York, 2001, 756-760, und das kürzlich vorgestellte PIRO-Konzept (Levy et al., Intensive Care Med. 29 (2003), 530-538 verwiesen, wo eine moderne Definition der Sepsis bzw. verwandten systemischen entzündlichen Erkrankungen dargestellt ist. Im Rahmen der vorliegenden Erfindung werden die Begriffe Sepsis bzw. entzündliche Erkrankung in Anlehnung an die Definitionen verwendet, wie sie den genannten Literaturstellen entnommen werden können.

Während in der Vergangenheit die positive Blutkultur (d.h. Nachweis eines Erregers im Blut) eine *conditio sine qua non* der Sepsisdiagnose darstellte, ist nach modernem Verständnis die inflammatorische Wirtsreaktion auf eine bakterielle, fungale oder virale Infektion ausschlaggebend für die Diagnose. Dem dargestellten Wandel des Verständnisses entsprechen Veränderungen der diagnostischen und therapeutischen Ansätze. So wurde der direkte Nachweis bakterieller Erreger durch Überwachung physiologischer Parameter und durch den Nachweis bestimmter endogener Substanzen wie das Prohormon Procalcitonin ergänzt.

Von den zahlreichen Mediatoren und Akutphase-Proteinen, von denen eine Beteiligung am Entzündungsgeschehen vermutet wird oder nachgewiesen ist, eignen sich für diagnostische und therapeutische Zwecke insbesondere solche, deren Auftreten sehr spezifisch für entzündliche Erkrankungen bzw. bestimmte Phasen einer entzündlichen Erkrankung ist, deren Konzentration oder enzymatische Aktivität sich drastisch und diagnostisch signifikant verändern sowie ebenjene, welche die für Diagnose oder therapeutische Überwachung erforderliche Stabilität und Halbwertszeit im Organismus aufweisen. Außerdem steht eine zuverlässige Assoziation von Krankheitsgeschehen (Entzündung, Sepsis) mit der Konzentration bzw. Aktivität des Mediators bzw. des Akutphase-Proteins im Vordergrund, ohne dass dessen Rolle im komplexen Ablauf der Entzündungsreaktion detailliert bekannt sein muss.

Die derzeit zur Verfügung stehenden Verfahren, welche zur Diagnose- und Therapiekontrolle der vorstehenden Erkrankungen und Krankheitszustände eingesetzt werden, weisen jedoch erhebliche Mängel hinsichtlich Sensitivität und Spezifität auf.

Somit liegt der vorliegenden Erfindung das technische Problem zugrunde, Marker bereitzustellen, die eine verbesserte und spezifischere Diagnose von Krankheitszuständen wie Sepsis, Sepsis-ähnlichen schweren Infektionen oder Gerinnungszuständen erlauben und damit auch verbesserte therapeutische Maßnahmen.

Die Lösung dieses technischen Problems wird durch die Bereitstellung der in den Patentansprüchen gekennzeichneten Ausführungsformen erzielt.

Im Verlauf der zu der vorliegenden Erfindung führenden Untersuchungen zeigte sich überraschenderweise, dass bei Patienten mit schwerer Sepsis oder septischen Schock sowie bei Patienten nach Operationen am offenen Herzen unter Verwendung eines kardiopulmonalen Bypasses die proteolytische Aktivität von ADAMTS-13 deutlich vermindert ist, somit die Konzentration/Aktivität dieses Faktors von hoher diagnostischer und möglicherweise therapeutischer Relevanz ist.

Der von-Willebrand-Faktor (vWF) ist ein adhäsives Glykoprotein, welches im Menschen in Endothelzellen und Megakaryozyten synthetisiert wird. Durch die Bildung von Dimeren und deren weitere Zusammenlagerung entstehen sehr lange Ketten mit mehr als 20 Primäreinheiten, die als Multimere bezeichnet werden und ein Molekulargewicht von bis zu 20.000 kDa erreichen können. Da jede Primäreinheit Bindungsstellen für subendotheliale Strukturen (Kollagen) einerseits und Thrombozyten andererseits besitzt, ergibt sich durch die Assoziation zu Multimeren ein Multiplikationseffekt für die Wirksamkeit des vWF. Nur vWF in Form langer Ketten kann seine Wirkung voll entfalten. Die Multimere werden zum Teil in den Endothelzellen gespeichert, aus denen sie nach deren Aktivierung freigesetzt werden und zirkulieren im Plasma in einer Konzentration von 5-10 mg/ml.

Aufgrund von zwei Funktionen spielt der vWF eine essentielle Rolle bei der Aufrechterhaltung der Hämostase: zum einen stabilisiert er als Carrierprotein den Blutgerinnungsfaktor VIII (FVIII), der ohne Bindung an vWF im Plasma sehr schnell u.a. durch aktiviertes Protein C proteolytisch inaktiviert werden würde (Fay et al., J. Biol. Chem. 266 (1991), S. 2172-2177). Weiterhin ist er an der Thrombozytenaggregation durch seine Wirkung als extrazelluläres Adapterprotein beteiligt: er vernetzt die verletzte Gefäßwand mit Thrombozyten sowie die Thrombozyten untereinander. Bedeutsam ist außerdem seine Affinität zu Kollagen, einer Komponente der extrazellulären Matrix, welche in intakten Gefäßen aufgrund des Monolayer aus Endothelzellen keinerlei Kontakt zum Blutstrom hat. In Folge einer Verletzung des Gefäßes resultiert jedoch die direkte Exposition zum Blutstrom, wobei vWF aufgrund seiner Bindungsaffinität zum freigelegten, ursprünglich subendothelialen Kollagen und Thrombozyten an der Bildung und Fixierung des initialen Thrombozytenagglomerates beteiligt ist.

Eine Protease, welche unter denaturierenden Bedingungen in Gegenwart von Barium- oder anderen mehrwertigen Metallionen aktiviert wird, spaltet aggregabile, hochmolekulare vWF-Multimere in Multimere kleinerer Größe und geringerem Molekulargewicht. Dieses Enzym wird als vWF-spaltende Protease (*vWF-cleaving protease, vWF-CP*), systematisch jedoch als ADAMTS-13 bezeichnet (Moake, N. Engl. J. Med. 347 (2002), S. 589) und reguliert die Funktion des vWF. Eine deutliche Aktivitätsverminderung dieser Protease und die dadurch auftretenden ungewöhnlich hochmolekularen vWF-Multimere stellen die pathophysiologische Grundlage einer Erkrankung dar, welche durch Mikrothrombosierung gekennzeichnet ist und als Thrombotisch-Thrombozytopenische Purpura (TTP) bezeichnet wird (Levy et al., Nature 413 (2001), 488-494). Die TTP-assoziierten hochmolekularen vWF-Multimere verschwinden unter adäquater Therapie. Dazu werden Plasmapräparate infundiert, welche aufgrund ihrer Herstellungsweise eine nachweisbare ADAMTS-13 Aktivität enthalten. Neben der kongenitalen Form der TTP ist eine Aktivitätsverminderung durch auto-inhibitorische Antikörper nachgewiesen.

Nach bisherigem Wissenstand sind Defizite in der proteolytischen Aktivität der ADAMTS-13 multifaktoriell bedingt. Zu den diskutierten Faktoren gehören neben polygenetischen Prädispositionen und Mutationen, welche mit Funktionseinschränkung bzw. -verlust des Proteins gekennzeichnet sind, Schwangerschaft, akute und chronische Entzündungen, Knochenmarks-Transplantationen, renale Insuffizienz und die Anwendung vasopressorischer Arzneimittelzubereitungen wie z. B. Vasopressin und dessen stabilisierten Analoga wie z.B. Desmopressin (*INN*). Der Quotient zwischen dem Antigengehalt und der ADAMTS-13 Aktivität kann als funktioneller Parameter zur Beschreibung der Gerinnungstendenz (TMA-Quotient = (vWF:Ag) : (ADAMTS-13)) angesehen werden und schwankt bei gesunden Referenzprobanden zwischen 0,3 und 1,8, kann jedoch bei Vorliegen einer der genannten pathophysiologischen Störungen wesentlich höhere Werte erreichen.

Die vorliegende Erfindung beruht auf Erkenntnissen, die mit einem speziell konzipierten analytischen Verfahren der immunologischen Proteinanalyse gewonnen wurden, das rein empirisch ist und in das, außer der Annahme, dass eine akute oder chronische Entzündungsreaktion in einer Aktivitätsveränderung der ADAMTS-13 deutlich werden könnte, keine zusätzlichen hypothetischen oder theoretischen Ansätze eingehen. Es zeigte sich, dass bei Patienten, bei denen aufgrund der klinischen Befunde eine Sepsis diagnostiziert war, mit hoher Sensitivität und Spezifität im Unterschied zu Gesunden veränderte funktionelle Eigenschaften des von-Willebrand-Faktors und eine Aktivitätsverminderung der ADAMTS-13 gefunden wurden, was dieses Protein ADAMTS-13 sowohl für die Diagnostik, Verlaufsbeobachtung als auch als therapeutisches Target geeignet erscheinen lässt. Die Beteiligung von ADAMTS-13 am Krankheitsgeschehen, welche bei entzündlichen Erkrankungen und Infektionen sowie Sepsis eine Rolle spielt, war bisher völlig unbekannt.

Die hierin beschriebenen experimentellen Befunde machen es somit erstmals möglich, eine veränderte ADAMTS-13 Aktivität und/oder veränderte funktionelle Eigenschaften des von-Willebrand-Faktors in biologischen Proben, insbesondere in Serum, Plasma, Gewebeproben und Körperflüssigkeiten mit hoher diagnostischer Aussagekraft neu zu bewerten. Somit können die ADAMTS-13 Aktivität und/oder veränderte funktionelle Eigenschaften des von-Willebrand-Faktors aufgrund der vorliegenden Ergebnisse als Parameter (Biomarker) zum diagnostischen Nachweis und zur Verlaufskontrolle von Entzündungen, Infektionen (insbesondere auch von systemischen Infektionen - Sepsis) und Störungen der Blutgerinnung dienen. Dabei kann die Bestimmung zur differentialdiagnostischen Früherkennung und zur Erkennung sowie für die Erstellung einer Verlaufsprognose, zur Beurteilung des Schweregrades und zur therapiebegleitenden Verlaufsbeurteilung von Sepsis, Infektion etc. erfolgen. Dabei wird bei einem derartigen Verfahren in einer Probe einer biologischen Flüssigkeit oder eines Gewebes eines Patienten die Aktivität der ADAMTS-13 und die genannten funktionellen Parameter direkt oder indirekt bestimmt und aus der festgestellten proteolytischen Aktivität und den Werten der genannten funktionellen Parameter und/oder aufgrund einer ebendiese Werte berücksichtigende Berechnungsmethode auf das Vorliegen einer Entzündung, einer schweren Infektion, einer Sepsis etc. geschlossen und das erhaltene Ergebnis mit dem Schweregrad der Erkrankung korreliert und die Behandlungsmöglichkeiten und/oder die Behandlungsaussichten abgeschätzt.

### Kurze Beschreibung der Figuren

### Figur 1: Zusammenfassung einzelner Parameter von elf Patienten mit schwerer Sepsis oder septischem Schock

Die Ergebnisse sind als Box- und Whiskers-Blots zusammengefasst; siehe dazu auch die weiteren Angaben in Beispiel 2.

### Figur 2: Darstellung der individuellen Tagesverläufe von 2 Patienten und der Nachweis von ungewöhnlich langen vWF Multimeren bei Patienten mit schwerer Infektion

Der Verlauf des vWF-Gehalts (ausgefüllte Kreise) und der ADAMTS-13 Aktivität (ungefüllte Quadrate) wurden von einem Nicht-Überlebenden und einem Überlebenden (beide mit schwerer Sepsis bzw. septischem Schock) gegenübergestellt; siehe dazu auch die weiteren Angaben in Beispiel 2.

Gegenstand der vorliegenden Erfindung ist somit ein Verfahren zur Diagnose einer Entzündung, Infektion und/oder Gerinnungsstörung, das dadurch gekennzeichnet ist, dass in einer Patientenprobe die Aktivität und/oder Konzentration von ADAMTS-13 bestimmt wird, wobei eine signifikant erniedrigte Aktivität und/oder Konzentration im Vergleich zu gesunden Personen ein Anzeichen für eine Entzündung, Infektion und/oder Gerinnungsstörung ist. Von den vorstehenden Krankheitszuständen sind auch Entzündungen, Verbrennungen, schwere Gewebsverletzungen, Geburtskomplikationen, Organ- und/oder Knochenmarkstransplantationen, SIRS und sepsisähnliche systemischen Infektionen umfasst.

Geeignete Verfahren zur Gewinnung einer Patientenprobe sind dem Fachmann bekannt. Bevorzugte Patientenproben sind Serum, Plasma, Gewebeproben oder Körperflüssigkeiten z.B. von Patienten, die an einer der hier genannten Erkrankungen leiden oder bei denen der Verdacht auf eine derartige Erkrankung oder eine Vorstufe für eine solche Erkrankung besteht. Der Nachweis von ADAMTS-13 kann direkt oder indirekt über gängige Verfahren erfolgen, wobei von der bekannten Aminosäuresequenz von ADAMTS-13 oder der Nukleinsäuresequenz des entsprechenden Gens ausgegangen wird (Levy, Nature 413 (2001), S. 488). Hinsichtlich des direkten Nachweises kann sich der Nachweis auf die Transkription (Nachweis der Konzentration der mRNA über gängige Verfahren) oder das ADAMTS-13-Protein selbst und dessen Splicing-Varianten beziehen, wobei der Proteinnachweis bevorzugt ist. Der hier verwendete Ausdruck "indirekt nachgewiesen" bezieht sich auf Nachweisverfahren, bei denen vorzugsweise eine Veränderung des Substrats von ADAMTS-13 untersucht wird, d.h., vWF. Wesentlichstes Kriterium zur Charakterisierung der vWF-Funktionalität auf molekularer Ebene stellt die Multimerenanalyse z.B. mittels Agarose-Gelelektrophorese dar, d.h. die Untersuchung der Spaltung hochmolekularer von-Willebrand-Faktor-Multimere durch ADAMTS-13 in Multimere mit kleinerem Molekulargewicht. Dabei ist eine annähernd gleichmäßige Verteilung der Multimere, welche aus bis zu 16 Einzelbausteinen (Monomeren) zusammengesetzt sind, ein Anzeichen dafür, dass die Aktivität der ADAMTS-13 im Normalbereich liegt. Jedoch ist das Auftreten von Multimeren, welche aus mehr als 16 Einzelbausteinen (Monomeren) zusammengesetzt sind sowie insgesamt eine Verschiebung des jeweiligen Anteils zu höhermolekulargewichtigen Monomeren ein Anzeichen dafür, dass die Aktivität der ADAMTS-13 im pathologischen Bereich liegt. Nach derzeitigem Wissensstand werden die vWF-Multimere wie folgt eingeteilt: niedermolekulare Multimere (ein bis vier Monomere), mittelgewichtige Multimere (5-9 Monomere) sowie hochmolekulare Multimere (mehr als 10 Monomere). Bei einer densitometrischen Auswertung einer gelelektrophoretischen Auftrennung ergibt sich beim gesunden Probanden folgende Verteilung (nachgestellt in Klammern Anteil bei einer verminderten ADAMTS-13 Aktivität zum Vergleich): niedermolekular 40-50% (< 40%), mittelgewichtig 30-40% (< 30%), hochmolekular 10-20%, jedoch keine Multimere, welche aus mehr als 16 Monomeren zusammengesetzt sind (> 20% sowie Nachweis vom Multimeren, welche aus mehr als 16 Monomeren zusammengesetzt sind) (Barrington, Vox. Sang. 76 (1999), S. 85). Die funktionale Aktivität des von-Willebrand-Faktors kann weiterhin durch die Bestimmung der Ristocetin-Kofaktor-Aktivität (vWF:RisCoF), der Collagenbindungs-Aktivität (CBA) und die Bestimmung der absoluten Antigenmenge (vWF:Ag) beschrieben werden. Für alle drei Parameter liegt der Bereich, welcher als unpathologisch angesehen wird, zwischen 70 und 150% einer Normalplasmakontrolle gesunder Probanden (Schneppenheim, in: Labor und Diagnose, Indikation und Bewertung von Laborbefunden für die medizinische Diagnostik (ed. L. Thomas), 5. erw. Auflage 2000, TH-Books Verlagsgesellschaft Frankfurt/Main, S. 628-631). Werte außerhalb dieses Normalbereichs, insbesondere erhöhte Werte, werden als pathologisch angesehen.

Ansonsten zählen zu den geeigneten Verfahren zur Bestimmung der proteolytischen Aktivität von ADAMTS-13 in der biologischen Probe beliebige bekannte Verfahren der Immundiagnostik oder verwandte Techniken, die zum Nachweis und zur Messung der Aktivität/Menge von ADAMTS-13 und/oder z.B. veränderter funktioneller Eigenschaften des von-Willebrand-Faktors unter Verwendung natürlich vorkommender oder mittels gentechnischer und/oder chemischer Verfahren hergestellter Substrate angewandt werden. Selbstverständlich ist vorzugsweise ein solcher Assay zur Aktivitätsbestimmung und Bestimmung veränderter funktioneller Eigenschaften zu verwenden, der die benötigte Empfindlichkeit im Bereich der beobachteten Veränderungen gewährleistet. Das Bestimmungsverfahren kann auch unter Verwendung von Protein-Chiptechnologie oder als Schnelltest (Point-of-Care-Test) zum Beispiel unter Verwendung spezifischer Antikörper beziehungsweise deren Fragmente und/oder unter Verwendung von spezifisch bindenden, zum Beispiel Kollagenbeschichteten Oberflächen, ausgeführt werden. Das als Substrat zu verwendende Protein kann dabei ein humanes oder tierisches Protein sein, das aus geeigneten natürlichen Quellen menschlichen oder tierischen Ursprungs angereichert oder isoliert wurde oder das rekombinant in vollständiger Form oder in Form eines Teilpeptides erzeugt wurde. Das Bestimmungsverfahren kann auch den direkten oder indirekten Nachweis ungewöhnlich langer vWF-Multimere, welche bei gesunden Normalprobanden nicht oder in signifikant niedrigerer Menge auftreten, beinhalten. Die dabei zu verwendende Matrix kann instrumentell oder visuell ausgewertet werden.

In einer bevorzugten Ausführungsform des erfindungsgemäßen Diagnoseverfahrens bezieht sich die Diagnose auf eine differentialdiagnostische Früherkennung, eine Beurteilung des Schweregrads, eine Verlaufsprognose oder eine therapeutische Verlaufsbeurteilung, wobei eine Erhöhung der Aktivität und/oder Konzentration von ADAMTS-13 im Vergleich zu einem früheren Wert ein Anzeichen für einen positiven therapeutischen Verlauf ist.

Die Bestimmung der Aktivität/Menge an ADAMTS-13 kann darüber hinaus auch als indikationsunabhängiger Diagnostikparameter dienen.

Die vorliegende Erfindung betrifft auch ein Verfahren zur Beurteilung der Wirkung von Wirkstoffkandidaten für die therapeutische Beeinflussung des Verlaufs einer Entzündung, Infektion und/oder Gerinnungsstörung, das dadurch gekennzeichnet ist, dass in einer Patientenprobe die Aktivität und/oder Konzentration von ADAMTS-13 direkt oder indirekt bestimmt wird, wobei eine signifikant erhöhte Aktivität und/oder Konzentration nach Verabreichung des Wirkstoffkandidaten ein Anzeichen für eine therapeutische Wirksamkeit des Wirkstoffkandidaten ist.

Vorzugsweise handelt es sich bei den diagnostizierbaren Erkrankungen um eine systemische Inflammationserkrankung, Sepsis, eine Sepsis-ähnliche schwere Infektion, und/oder Gerinnungsstörung.

Weitere mit dem Verfahren der vorliegenden Erfindungen anhand von ADAMTS-13 direkt oder indirekt diagnostizierbar Erkrankungen sind Verbrennungen, schwere Gewebsverletzungen, Geburtskomplikationen, Organ- und/oder Knochenmarkstransplantationen, außerdem lokale oder generalisierte Infektionen, bakterielle, virale, parasitäre und durch Pilze verursachte Infektionen und deren Folgeerkrankungen wie das Systemische Inflammations-Response-Syndrom (SIRS), septischer Schock, einfaches oder mehrfaches Organversagen, Meningitis, Waterhouse-Friderichsen-Syndrom, Purpura anaphylactoides und deren Unter- und Mischformen, Erkrankungen des rheumatischen Formenkreises, Arthrose und Osteoporose, chronisch entzündliche Darmerkrankungen wie Morbus Crohn, Colitis ulcerosa sowie deren Mischformen, metabolisch-endokrine Störungen wie unter anderem Diabetes mellitus, Atherosklerose, koronare Herzkrankheit, Gerinnungsstörungen wie z. B.
Verbrauchkoagulopathie, Kasabach-Merritt-Syndrom, oder im Rahmen einer Thrombozytopenie, Morbus Werlhof, Schwangerschafts- und Geburtskomplikationen wie z.B. das HELLP-Syndrom, Vasopathien, Vaskulitiden, Morbus Osler, Autoimmunerkrankungen wie z.B. allergischen Reaktionen oder systemischer Lupus erythematodes, Bindegewebserkrankungen, renale, pulmonale, myokardiale, pankreatische, lienale sowie hepatische Insuffizienz unterschiedlicher Genese, chronisch obstruktive und/oder restriktive Lungenerkrankungen, Mukoviszidose, Zustände nach extrakorporaler Zirkulation, Zustände nach Organtransplantation, posttraumatische Stressreaktionen, Polytrauma, postoperative Stressreaktionen, akutes Atemwegssyndrom (acute respiratory distress syndrome, ARDS), Zustände während oder nach mechanischer Ventilation, benigne, maligne und metastasierende Erkrankungen wie u. a. Hämangiome, Zustände nach und/oder während nuklearmedizinischer, cytostatischer sowie immunsuppresiver Therapie, Medikamenten-induzierter ADAMTS-13-Mangel wie u.a. nach Verabreichung von Thrombozytenaggregationshemmern, insbesondere vom Thienopyridin-Typ, in Folge einer Verabreichung von Vasopressin oder dessen stabilisierten Analoga, im Rahmen eines Leberersatzverfahrens, im Rahmen von Störungen im hämatopoetischen oder retikulo-endothelialen System, neurodegenerative Erkrankungen wie Morbus Alzheimer, vaskuläre Demenz wie u.a. Morbus Binswanger, hämorrhagischer Insult, Epilepsie, Polyneuropathie, Multiple Sklerose, Anwendung im Rahmen einer antiviralen Chemotherapie z. B. bei Infektionen mit dem HI-Virus, sämtlicher Hepatitis- sowie Herpes-Viren.

In einer besonders bevorzugten Ausführungsform des erfindungsgemäßen Verfahrens wird die ADAMTS-13 Konzentration daher dadurch bestimmt, dass man die Patientenprobe mit einem anti-ADAMTS-13-Antikörper oder einem Fragment davon in Berührung bringt und sodann die Bindung des anti-ADAMTS-13-Antikörper oder des Fragments davon an ADAMTS-13 bestimmt.

Dabei kann es sich bei den dafür geeigneten Antikörpern um monoklonale, polyklonale oder synthetische Antikörper oder Fragmente davon handeln. In diesem Zusammenhang bedeutet der Begriff "Fragment" alle Teile des monoklonalen Antikörpers (z.B. Fab-, Fv- oder "single chain Fv"-Fragmente), welche die gleiche Epitopspezifität wie der vollständige Antikörper aufweisen. Die Herstellung solcher Fragmente ist dem Fachmann bekannt. Vorzugsweise handelt es sich bei den erfindungsgemäßen Antikörpern um monoklonale Antikörper. Die erfindungsgemäßen Antikörper können gemäß Standardverfahren hergestellt werden, wobei vorzugsweise ADAMTS-13 oder ein synthetisches Fragment davon als Immunogen dienen. Dieses Polypeptid oder Peptid bzw. das Fragment davon können z.B. durch Gewinnung des entsprechenden Gens, Klonierung und rekombinante Expression erzeugt werden. Verfahren zur Gewinnung monoklonaler Antikörper sind dem Fachmann bekannt.

In einer noch mehr bevorzugten Ausführungsform des erfindungsgemäßen Verfahrens wird die ADAMTS-13 Konzentration über einen primären monoklonalen anti-ADAMTS-13-Antikörper und einen im Handel erhältlichen sekundären Biotin-konjugierten Antikörper und Streptavidin-Peroxidase bestimmt.

In einer weiteren bevorzugten Ausführungsform des erfindungsgemäßen Verfahrens ist die Patientenprobe immobilisiert, z.B. als Paraffinschnitt. Die Patientenprobe kann auch an die Wand einer Plastikschale über übliche Verfahren so absorbiert sein, dass ADAMTS-13 seine Bindungsspezifität, etwa für einen spezifischen Antikörper, nicht verliert.

In einer weiteren bevorzugten, alternativen Ausführungsform des erfindungsgemäßen Verfahrens sind der anti-ADAMTS-13-Antikörper oder das Fragment davon immobilisiert, d.h., dass er z.B. an der Wand einer Plastikschale absorbiert ist ohne seine Bindungsspezifität für ADAMTS-13 zu verlieren.

Der Nachweis der Bindung des Antikörpers kann über übliche Verfahren erfolgen, z.B. Western-Blot, ELISA, Radioimmunassay (RIA) etc., wobei RIA und ELISA bevorzugt sind.

In einer weiteren bevorzugten, alternativen Ausführungsform des erfindungsgemäßen Verfahrens erfolgt die Bestimmung von ADAMTS-13 indirekt dadurch, dass das das Molekulargewicht von vWF-Multimeren bestimmt wird, wobei das Vorhandensein von vWF-Multimeren mit im Vergleich zu Normalpersonen erhöhtem Molekulargewicht diagnostisch relevant ist. Zum Nachweis können z.B. anti-vWV-Antikörper verwendet werden, wobei zur weiteren Ausgestaltung dieses Verfahrens die vorstehend bezüglich ADAMTS-13 gemachten Angaben prinzipiell gelten. Die MultimerenAnalyse kann auch mittels Gelelektrophorese nach gängigen Verfahren erfolgen. Diagnostisch relevant ist das Auftreten von vWF-Multimeren ungewöhnlicher Größe, die bei gesunden Normalpersonen nicht oder lediglich in signifikant geringerer Menge nachweisbar sind (Auftreten von Multimeren mit mehr als 16 Monomeren, Anteil der höhermolekularen Multimere bei einer densitometrischen Auswertung > 20%).

Die vorliegende Erfindung stellt auch Kits bereit, die für die erfindungsgemäßen Diagnoseverfahren von Nutzen sind und vorzugsweise (a) einen anti-ADAMTS-13-Antikörper oder ein Fragment davon enthalten und außerdem ADAMTS-13 oder einen bindungsaktiven Teil davon zur Kontrolle und/oder (b) einen anti-vWF-Antikörper oder ein Fragment davon enthalten und außerdem vWF oder einen bindungsaktiven Teil davon. Der Ausdruck "bindungsaktiver Teil" bezeichnet ein Fragment, das mit dem Antikörper reagiert, mit dem auch das Gesamt-Molekül reagiert. Je nach Ausgestaltung des Kits kann der Antikörper an eine weitere Einheit konjugiert sein, beispielsweise eine Markierung und/oder er kann auf einem festen Träger (Substrat) immobilisiert sein. Der Kit kann auch einen zweiten Antikörper zum Nachweis von ADAMTS-13/Antikörper-Komplexen bzw. vWF/Antikörper-Komplexen enthalten. Der Antikörper oder das Fragment davon können frei vorliegen oder an einen festen Träger immobilisiert sein, beispielsweise eine Plastikschale, ein Teströhrchen, eine Mikrotiterplatte, ein Teststäbchen etc. Der Kit kann auch Anleitungen enthalten, die die Verwendung des Antikörpers oder des Fragments davon in einem Assay zum Nachweis der bestimmten Krankheitszuständen beschreiben. Der Kit kann weiterhin geeignete Reagenzien zum Nachweis von Markierungen oder zur Markierung positiver und negativer Kontrollen, Waschlösungen, Verdünnungspuffer etc. enthalten.

Da zwischen Überlebenden und Nicht-Überlebenden z.B. einer schweren Sepsis bzw. eines septischen Schocks deutliche Unterschiede in der ADAMTS-13-Aktivität sowie der funktionellen Eigenschaften des vWF nachgewiesen werden konnten, ist die Verabreichung von arzneilichen Zubereitungen mit nachgewiesener ADAMTS-13 Aktivität im Sinne einer Replacement-Therapie wirksam, so dass der hierin berichtete diagnostische Befund einer verminderten ADAMTS-13 Aktivität auch neue therapeutische Möglichkeiten erschließt. Für derartige Zwecke können auch Arzneimittel verwendet werden, welche als eigentlichen Wirkstoff ein Protein, ein Teilpeptid oder niedermolekulare Verbindungen enthalten, welche in Analogie zu ADAMTS-13 die funktionellen Eigenschaften des vWF verändern, für eine Verabreichung am Patienten zusammen mit einem pharmazeutischen Träger enthalten.

Zur Schaffung neuartiger therapeutischer Ansätze bei den genannten Erkrankungen stellt folglich die Beeinflussung des Krankheitsgeschehens durch die Aufhebung des Mangels an proteolytischer Aktivität einen zentralen Aspekt dar. Dies kann z.B. durch die Anwendung arzneilicher Zubereitungen erreicht werden, von denen bekannt ist, dass sie eine nachweisbare ADAMTS-13 Aktivität enthalten oder induzieren, und/oder in denen aus geeigneten natürlichen Quellen menschlichen oder tierischen Ursprungs ADAMTS-13 angereichert oder isoliert wurde, und/oder in denen ADAMTS-13 rekombinant in vollständiger Form oder in Form eines Teilpeptides, eines Peptidanalogons, eines chemisch oder enzymatisch veränderten Peptides oder einer Splicingvariante erzeugt wurde, und/oder insbesondere bei letztgenannten Zubereitungen in Form eines Adduktes der ADAMTS-13 ausgehend von der vollständigen Form oder ausgehend von einem Teilpeptid, einem Peptidanalogons oder einer Splicingvariante beispielsweise zur Beeinflussung der Plasmahalbwertszeit durch Veränderung des Glykolysierungs- und oder Phosphorylierungsmusters oder auch in einer durch pharmazeutische Hilfsstoffe, z. B. Polyethylenglykolreste substituierten Form oder andere geeignete Verfahren, oder in Form einer zur extra-korporalen Anwendung immobilisierten Zubereitung, welche ADAMTS-13 in vollständiger Form oder in Form eines Teilpeptides, eines Peptidanalogons, einer Splicingvariante oder eines Adduktes gleichwelcher Herkunft enthält, erfolgen. Die erfindungsgemäßen Zubereitungen können mit dem Fachmann bekannten Verfahren hergestellt werden. Ihre therapeutische Eignung, d.h. die Erhöhung der proteolytischen Aktivität in humanem Plasma bzw. die Beeinflussung der Größe der vWF-Multimere kann über bekannte Verfahren bestimmt werden Somit betrifft die vorliegende Erfindung auch die Verwendung von ADAMTS-13, eines biologisch aktiven Derivats oder aktiven Fragments davon, einer diese codierenden Nukleinsäure oder einer Verbindung, die die Transkription und/oder Translation des ADAMTS-13 Gens erhöht, zur Herstellung eines Arzneimittels (a) zur Behandlung und/oder Prophylaxe von mit einer erniedrigten Aktivität und/oder Konzentration von ADAMTS-13 in Zusammenhang stehenden Erkrankung, wobei diese Erkrankung z.B. eine Entzündung, Infektion oder Gerinnungsstörung ist oder (b) zur adjuvanten Therapie, die die Wechsel- oder Nebenwirkungen anderer Wirkstoffe mit Einfluss auf die Hämostase und/oder Blutgerinnung vermindern oder verhindert. Dabei sind insbesondere die Wechselwirkungen zwischen Blutgerinnungsfaktoren und dem vWF von erheblicher Bedeutung; sowohl FVIII als auch dessen aktivierte Form, Faktor VIIIa, werden in Gegenwart von vWF vor dem proteolytischen Abbau durch aktiviertes Protein C geschützt. Bei diesem Prozeß ist die Menge und Funktionalität von vWF von bestimmendem Einfluß. Daher ergeben sich durch eine gezielte Beeinflussung der Interaktion zwischen vWF, vWF-modifizierenden Enzymen (z.B. ADAMTS-13), der Verabreichung von Protein C (z.B. im Rahmen einer Therapie einer schweren Sepsis) und der Stabilisierung des Faktors VIII neue therapeutische Optionen, welche bei der Diagnose und Therapie der genannten Erkrankungen, insbesondere von systemischen Entzündungen infektiöser Ätiologie und Sepsis, von Bedeutung sein können.

Der hier verwendete Ausdruck "Derivat" betrifft alle Formen von ADAMTS-13, die sich von der natürlichen Form durch Aminosäureaustausche und/oder Aminosäuremodifikationen unterscheiden, aber noch biologisch aktiv sind. Dazu zählen z.B. Addukte, posttranslational oder chemisch modifizierte Proteine, z.B. Proteine, die eine Veränderung des Glykolysierungs- und/oder Phosphorylierungsmusters aufweisen, oder Splicingvarianten.

Die Bestimmung der ADAMTS-13 Aktivität und/oder der veränderten funktionellen Eigenschaften des vWF können jedoch auch in der präventiven Medizin, insbesondere von Gerinnungsstörungen und von systemischen Entzündungen infektöser Ätiologie und Sepsis und der obigen weiteren Erkrankungen, verwendet werden.

Die Arzneimittel für die vorstehende Verwendung enthalten vorzugsweise zusätzlich einen pharmazeutisch verträglichen Träger. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intra-arterielle, intramuskuläre, subkutane, intramedulläre, intrathekale, intraventrikuläre, intravenöse, intraperitoneale oder intranasale Verabreichung. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, der Art der Verabreichung etc..

Für die Gentherapie werden die ADAMTS-13 codierenden DNA-Sequenzen in einen für die Gentherapie geeigneten Vektor insertiert, beispielsweise unter Kontrolle eines gewebespezifischen Promotors, und in die Zellen eingeschleust. Ein für die Gentherapie geeigneter Vektor ist vorzugsweise ein Virus, beispielsweise ein Adenovirus, Vaccinia-Virus oder Adenovirus. Besonders bevorzugt sind Retroviren. Beispiele für geeignete Retroviren sind MoMuLV, HaMuSV, MuMTV, RSV oder GaLV. Für die Gentherapie geeignete Vektoren sind außerdem in WO 93/04701, WO 92/22635, WO 92/20316, WO 92/19749 und WO 92/06180 offenbart. Für Zwecke der Gentherapie können die ADAMTS-13 codierenden DNA-Sequenzen auch in Form von kolloidalen Dispersionen zu den Zielzellen transportiert werden. Dazu zählen beispielsweise Liposomen oder Lipoplexe (Mannino et al., Biotechniques 6 (1988), 682). Diese Gentherapie wird vorzugsweise für die Erhöhung des ADAMTS-13 Spiegels durchgeführt.

Die folgenden Beispiele erläutern die Erfindung.

### Beispiel 1

### Allgemeine Verfahren

### (A) Bestimmung der ADAMTS-13 Aktivität

Zur Bestimmung der ADAMTS-13 Aktivität wurde Citratplasma von Patienten bzw. gesunden Probanden bei 37°C im Wasserbad für 10 Minuten aufgetaut. Zur Erstellung einer Standardkurve wurde die angegebene Menge Plasmapool von 45 gesunden Freiwilligen mit Harnstofflösung (1,5 M) wie in Tabelle 1 beschrieben verdünnt.

**Tabelle 1**

| ADAMTS-13 in % | Aktivität Plasmapool in µl | 1,5M Harnstoff |
|---|---|---|
| 200 | 40 | 160 |
| 100 | 40 | 360 |
| 75 | 10 | 123 |
| 50 | 10 | 190 |
| 40 | 10 | 240 |
| 33 | 10 | 290 |
| 20 | 10 | 490 |
| 10 | 10 | 990 |
| 5 | 500µl der 10% Verdünnung | 500 |
| 2,5 | 500µl der 5% Verdünnung | 500 |

Die Patientenproben wurden seriell verdünnt (1:10, 1:20, 1:40). Anschließend wurden sowohl von den Verdünnungen der Standardreihe als auch von den Patientenverdünnungen je 50 µl in Eppendorf-Röhrchen überführt. Zum Abbau des im Plasmapool und in den Patientenproben vorhandenen vWF wurden zu jeder Probe und Pufferkontrolle 5 µl Bariumchlorid zur Aktivierung der ADAMTS-13 gegeben und für 30 min bei 37 °C inkubiert.

Als Substrat für die ADAMTS-13 dienten je 100 µl rekombinanter vWF (Schneppenheim, Blood 97 (2001), S. 2059) - Antigengehalt 100 % im Vergleich zu Normalplasmapool (NPP) - (2 h 37°C, Reaktionsbeendigung mittels Natriumsulfat). Nach Zentrifugation (2 min, 13.000 x g) und Verdünnung in Probenverdünnungspuffer erfolgte eine Inkubation in Kollagen-III-beschichteten Cova-Link Streifen (Southern Biotechnologies, Bezug über Fa. BIOMOL, Hamburg, Deutschland) (Vorbereitung: 100 µl Kollagen III (1,5 µg/ml) werden 1 h inkubiert, ausgeschlagen und mit 2,5 % BSA für 15 Minuten blockiert). Innerhalb der zweistündigen Inkubation lagern sich aus der Proteolyse resultierende vWF-Fragmente aufgrund ihrer unterschiedlichen Größe und der daraus resultierenden Affinität zu Kollagen III an. Nach dreimaligem Waschen mit PBS erfolgte die Detektion mittels Peroxidasemarkierten Anti-vWF-Antikörper (Fa. DAKO, Hamburg, Deutschland)(Verdünnung 1:4000, Inkubation 1 h bei Raumtemperatur). Die Visualisierung erfolgte mittels kommerziellem ELISA-Detektionskit (TMB, Biorad) und Vermessung im Mikroplattenleser bei 450 nm. Die Auswertung erfolgte durch Berechnung der ADAMTS-13 Aktivität aufgrund der Standardkurve und Kalkulation der einzelnen Patientenverdünnungen.

### (B) Bestimmung der RisCof-Aktivität

Die RisCof-Aktivität wurde mittels dem Fachmann bekannter immunologischer Standardmethoden bestimmt (Dade-Behring Gerinnungsanalyzer). (Coller, von-Willebrand-Disease. Haemostasis and Thrombosis, Colman et al., (eds.), Lippincott, Philadelphia (1987), S. 60-96).

### (C) Bestimmung der CBA und vWF:Ag

Die Collagen-Bindungsaktivität (CBA) und die Konzentration an vWF-Antigen (vWF:Ag) wurden ebenfalls mittels immunologischer Methoden bestimmt. Zur Bestimmung der Affinität des vWF gegenüber Kollagen wurden 96 Well Platten (Nunc-Immunoplate; Maxisorp Surface) mit Kollagen I beschichtet (6 µg/ml). Parallel dazu wurde zur Bestimmung der gesamten vWF-Proteinmenge eine 96 Well Platte (Nunc-Immunoplate; Maxisorp Surface) mit primärem Antikörper (Fa. DAKO, Hamburg, Deutschland) beschichtet (5,7 µg/ml). Nachfolgende Schritte erfolgen für beide Methoden in gleicher Weise. Zur Erstellung der Standardkurve wurde das in Tabelle 2 gezeigte Schema zugrunde gelegt.

**Tabelle 2**

| **vWF:Ag bzw. CBA** | **NPP (1: vorverd.)** | **10 PVP** |
|---|---|---|
| 100% | 100µl | 400µl |
| 50% | 100µl | 900µl |
| 25% | 50µl | 950µl |
| 12,5% | 25µl | 975µl |
| 6,25% | 25µl | 1975µl |
| 3,13% | 500µl der 6,25 % | 500µl |
| 1,5% | 500µl der 3,13 % | 500µl |

Die bei -80°C gelagerten Citratplasmaproben wurden bei 37°C für 10 min im Wasserbad aufgetaut. Jede Patientenprobe wurde 1:10 mit Probenverdünnungs-puffer (PVP) vorverdünnt und in Abhängigkeit vom zu erwartenden vWF:Ag Gehalt weiterverdünnt. Je 100 µl wurden als Doppelbestimmung auf die mit Kollagen bzw. Antikörper beschichtete Mikrotiterplatte aufgetragen. Die Platten inkubierten bei 37°C für 90 min im wasserdampfgesättigten Brutschrank. Nach dreimaligem Waschen erfolgte der Nachweis mittels Peroxidase-konjugiertem Antikörper (Fa. DAKO, Hamburg, Deutschland)(Verdünnung 1:2000) und Inkubation bei 37°C für 90 min im Brutschrank. Nach dreimaligem Waschen der Platten wurden diese mit TMB visualisiert und vermessen (Details s.o.).

### (D) vWF-Multimerenanalyse

Die Multimerenanalyse des von-Willebrand-Faktors wurde nach einer publizierten Methode durchgeführt (Barington and Kaersgaard, Vox Sang.76 (1999), 85-89).

### Beispiel 2 Verminderte ADAMTS-13 Aktivität in Patienten mit schwerer Sepsis oder septischem Schock

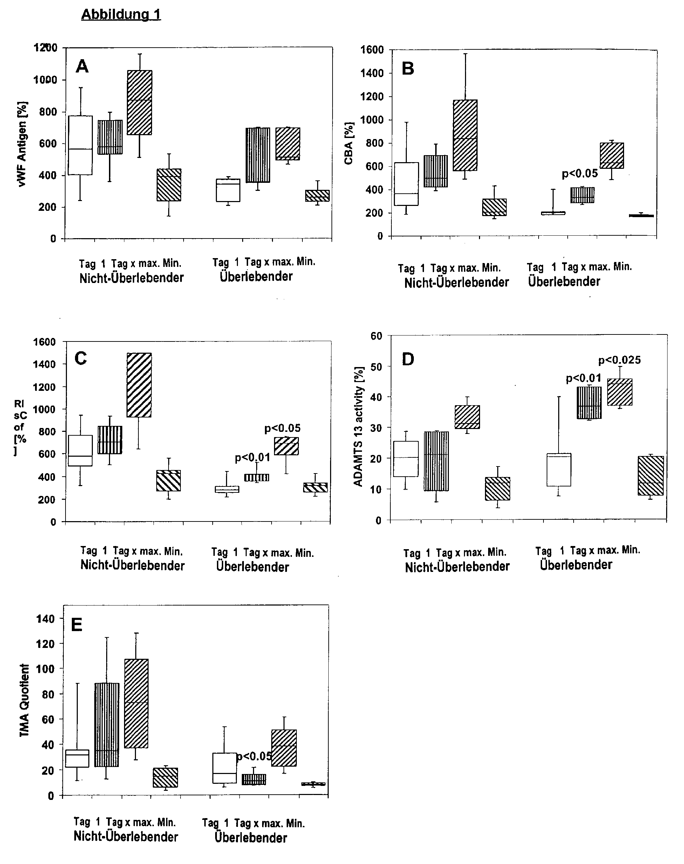

In Figur 1 sind einzelne Parameter von elf Patienten mit schwerer Sepsis oder septischem Schock in Form von Box- und Whiskers-Blots zusammengefasst. Um einen Überblick über den Tagesverlauf der analysierten Parameter vWF:Ag (**A**), CBA (**B**), RisCof (**C**), ADAMTS-13 Aktivität (**D**) im Plasma der gesamten Kohorte während des Aufenthaltes auf der Intensivstation zu erhalten, wurden alle Messwerte der Patienten mit schwerer Sepsis oder septischem Schock am Tag der Aufnahme (day1) und am Tag der Verlegung bzw. des Todes (day x) gegenübergestellt. In gleicher Weise wurde die Amplitude im Tagesverlauf aufgezeigt, indem alle minimalen und maximalen Werte im jeweiligen Patientenverlauf dargestellt wurden, wobei jeweils zwischen Überlebenden (*Survivors,* n=5) und Nicht-Überlebenden (*Non-Survivors,* n=6) unterschieden wurde. Der berechnete Quotient zwischen dem vWF-Antigengehalt und der ADAMTS-13 Aktivität ist als sogenannter TMA-Quotient in (**E**) dargestellt. Als Vergleich wurden Referenzwerte von 17 klinisch unauffälligen Probanden (25,7 ± 3,8 Jahre) bestimmt: vWF:Ag Gehalt 101,1 ± 39,6 %, CBA 112,7 ± 55,2 %, RisCof 156,8 ± 25,7 %, ADAMTS-13 Aktivität 112,0 ± 24,6 %, TMA-Quotient 0,94 ± 0,42.

Der vWF:Ag der kritisch kranken Patienten war signifikant im Vergleich zum gesundem Kontrollkollektiv erhöht (p<0,0001), jedoch ergaben sich keine signifikanten Differenzen innerhalb der Subgruppen Überlebende/Nicht-Überlebende (Bsp: Median Überlebende letzter Studientag: 476 % vs. Nichtüberlebende 580 %). Die Bestimmung der Collagen-Bindungsaktivität ergab ebenfalls eine signifikante Erhöhung im Patientenkollektiv gegenüber gesunden Kontrollen (p<0,001), am letzten Studientag ist eine signifikante Differenz zwischen den Subgruppen nachweisbar (328 % vs. 497%, p<0,05). Ähnliche Ergebnisse wurden bei der Bestimmung des Ristocetin-Cofaktors erhalten. Insgesamt sind die Werte der kritisch Kranken signifikant erhöht (p<0,0001), die Subgruppenanalyse offenbart signifikante Unterschiede am letzten Studientag (414,0 % vs. 703,5 %, p< 0,01) sowie im Maximalwert des Tagesverlaufs (693,0 % vs. 1496,5%, p<0,05). Die proteolytische Aktivität der ADAMTS-13 war beim Vergleich des Patientenkollektives mit gesunden Kontrollen signifikant vermindert (p<0,0001), es ergaben sich signifikante Differenzen am letzten Studientag (36,7 % vs. 21,2 %, p<0,01) sowie im Maximalwert des Tagesverlauf (44,0 % vs. 31,2, p < 0,025). Weiterhin war der TMA-Quotient als Maß für das Risiko der Entwicklung einer Thrombotischen Mikroangiopathie bei allen kritisch Kranken signifikant erhöht (p<0,001), auch ergibt sich eine signifikante Differenz am letzten Studientag zwischen Überlebenden und Nicht-Überlebenden (10,9 vs. 35,2, p<0,05). Statistisch signifikante Unterschiede zwischen den Subgruppen und das Signifikanzniveau wurden mit dem Wilcoxon-Test (Trampisch, Windeler, (eds) Ehle, Lange: Medizinische Statistik, 2. Auflage, 2000, Springer, Berlin, Heidelberg, New York, S. 238 ff.), zwischen Patienten und gesunden Kontrollen mittels Mann-Whitney *U*-Test berechnet.

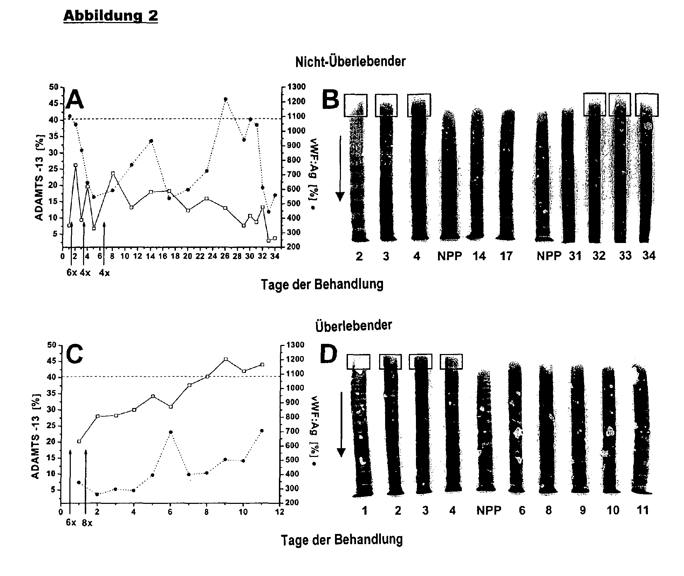

In Figur 2 sind individuelle Tagesverläufe von zwei Patienten und der Nachweis von ungewöhnlich langen vWF-Multimeren bei Patienten mit schwerer Infektion dargestellt. Der Verlauf des vWF:Ag Gehalts (ausgefüllte Kreise) und der ADAMTS-13 Aktivität (ungefüllte Quadrate) wurden von einem Nicht-Überlebenden und einem Überlebenden, beide mit schwerer Sepsis bzw. septischem Schock, gegenübergestellt: beim Nicht-Überlebenden (**A**) lassen sich am Studienbeginn deutlich verminderte ADAMTS-13-Werte (um ca. 10%) nachweisen, welche im Studienverlauf zwar alternieren, jedoch keine Tendenz zum Anstieg erkennen lassen. Der wellenförmige Verlauf der ADAMTS-13 Aktivität wird durch die Gabe von Fresh-Frozen-Plasma-Präparaten hervorgerufen, wobei der Zeitpunkt und die Anzahl an verabreichten Einheiten durch Pfeile angedeutet ist. Im Gegensatz (**C**) dazu ist der Überlebende - ausgehend von einer Aktivität von ca. 20% - durch einen steten Anstieg auf einen als nicht pathologisch anerkannten Wert am Verlegungstag von über 40% (gestrichelte Linie) charakterisiert. An ausgewählten Tagen lassen sich durch die Multimerenanalyse (**B, D**) pathologische ultralange vWF Multimere nachweisen. Zum Vergleich wurde gepooltes Plasma von gesunden, ausgewählten Probanden (n=45) aufgetragen, wobei man unter physiologischen Umständen zwischen den Banden 1-5 (low molecular vWF), 6-10 (intermediate molecular vWF) und > 11 unterscheidet (high molecular vWF). Aufgetragen wurde jeweils ein äquivalenter vWF:Ag Gehalt von 5-10 %. Die Präsenz der ultralangen vWF Multimere ist durch Rahmen gekennzeichnet und mit einer deutlich verminderten ADAMTS-13 Aktivtät assoziert. Beim Nicht-Überlebenden lassen sich zu Studienbeginn und präfinal ultralange vWF-Multimers nachweisen, hingegen verschwinden diese beim Überlebenden parallel zur ansteigenden ADAMTS-13-Aktivität.

## Patentansprüche

**1.** Verfahren zur Diagnose einer Entzündung, Infektion und/oder Gerinnungsstörung, **dadurch gekennzeichnet, dass** in einer Patientenprobe die Aktivität und/oder Konzentration von ADAMTS-13 direkt oder indirekt bestimmt wird, wobei eine signifikant erniedrigte Aktivität und/oder Konzentration im Vergleich zu gesunden Personen ein Anzeichen für eine Sepsis, Sepsisähnliche schwere Infektion und/oder Gerinnungsstörung ist.

**2.** Verfahren nach Anspruch 1, wobei die Diagnose eine differentialdiagnostische Früherkennung, eine Beurteilung des Schweregrads, eine Verlaufsprognose oder eine therapeutische Verlaufsbeurteilung ist, wobei eine Erhöhung der Aktivität und/oder Konzentration von ADAMTS-13 im Vergleich zu einem früheren Wert ein Anzeichen für einen positiven therapeutischen Verlauf ist.

**3.** Verfahren zur Beurteilung der Wirkung von Wirkstoffkandidaten für die therapeutische Beeinflussung des Verlaufs einer Entzündung, Infektion und/oder Gerinnungsstörung, **dadurch gekennzeichnet, dass** in einer Patientenprobe die Aktivität und/oder Konzentration von ADAMTS-13 direkt oder indirekt bestimmt wird, wobei eine signifikant erhöhte Aktivität und/oder Konzentration nach Verabreichung des Wirkstoffkandidaten ein Anzeichen für eine therapeutische Wirksamkeit des Wirkstoffkandidaten ist.

**4.** Verfahren nach einem der Ansprüche 1 bis 3, wobei die ADAMTS-13 Konzentration dadurch bestimmt wird, dass man die Patientenprobe mit einem anti-ADAMTS-13-Antikörper oder einem Fragment davon in Berührung bringt und sodann die Bindung des anti-ADAMTS-13-Antikörper oder des Fragments davon an ADAMTS-13 bestimmt.

**5.** Verfahren nach Anspruch 4, wobei die ADAMTS-13 Konzentration über einen primären monoklonalen anti-ADAMTS-13-Antikörper und einen sekundären Biotin-konjugierten Antikörper und Streptavidin-Peroxidase bestimmt wird.

**6.** Verfahren nach einem der Ansprüche 1 bis 5, bei dem die Patientenprobe immobilisiert ist.

**7.** Verfahren nach Anspruch 4 oder 5, bei dem der anti-L1-Antikörper oder das Fragment davon immobilisiert sind.

**8.** Verfahren nach einem der Ansprüche 4 bis 7, das als Radioimmunassay oder ELISA durchgeführt wird.

**9.** Verfahren nach einem der Ansprüche 1 bis 3, wobei die indirekte Bestimmung dadurch erfolgt, das das Molekulargewicht von vWF-Multimeren bestimmt wird, wobei das Vorhandensein von vWF-Multimeren mit im Vergleich zu Normalpersonen erhöhtem Molekulargewicht diagnostisch relevant ist.

**9.** Diagnostischer Kit zur Durchführung des Verfahrens nach einem der Ansprüche 1 bis 9, der (a) einen anti-ADAMTS-13-Antikörper oder ein Fragment davon und außerdem ADAMTS-13 oder einen bindungsaktiven Teil davon enthält und/oder (b) einen anti-vWF-Antikörper oder ein Fragment davon und außerdem vWF oder einen bindungsaktiven Teil davon.

**10.** Verwendung von ADAMTS-13, eines biologisch aktiven Derivats oder aktiven Fragments davon, einer diese codierenden Nukleinsäure oder einer Verbindung, die die Transkription und/oder Translation des ADAMTS-13 Gens erhöht, zur Herstellung eines Arzneimittels (a) zur Behandlung und/oder Prophylaxe von mit einer erniedrigten Aktivität und/oder Konzentration von ADAMTS-13 in Zusammenhang stehenden Erkrankung oder (b) zur adjuvanten Therapie, die die Wechsel- oder Nebenwirkungen anderer Wirkstoffe mit Einfluss auf die Hämostase und/oder Blutgerinnung vermindern oder verhindert.

**11.** Verwendung nach Anspruch 10, wobei die Erkrankung eine Entzündung, Infektion oder Gerinnungsstörung ist.
